(19)

**Europäisches Patentamt**

**European Patent Office**

**Office européen des brevets**

(11) **EP 4 510 138 A1**

(12) **EUROPEAN PATENT APPLICATION**

(43) Date of publication:
19.02.2025 Bulletin 2025/08

(51) International Patent Classification (IPC):
*G16C 20/30* (2019.01)

(21) Application number: 24153674.7

(22) Date of filing: 24.01.2024

(52) Cooperative Patent Classification (CPC):
G16C 20/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 14.08.2023 IN 202321054496

(71) Applicant: **Tata Consultancy Services Limited Maharashtra (IN)**

(72) Inventors:
• **RAMAMURTHI, NARAYANAN**
  **600 113 Chennai (IN)**
• **DAS, SHYAM SUNDAR**
  **751024 Bhubaneswar (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **ESTIMATION OF PHARMACOKINETIC (PK) PARAMETERS OF DRUG CANDIDATES USING UNIVERSAL PK PARAMETERS BOUNDS**

(57) Developability of a drug candidate is decided based on the Pharmacokinetic (PK) and Pharmacodynamic (PD) parameters of the drug candidate under investigation. Present disclosure provides systems and methods that are implemented using universal PK parameters' bounds and optimization technique(s) to produce robust and optimized set of PK parameters. More specifically, the system and method for estimating optimized set of PK parameters by a) creating universal parameter bounds, b) performing logical operations on universal PK parameters' bounds to create multiple bound combinations c) computing a performance threshold for residual sum of squares (RSS) d) performing global optimization to estimate globally optimized set of PK parameters act as initial PK parameters and e) performing local optimization of initial PK parameters to estimate locally optimized set of PK parameters, the best PK parameters that can used for assessing the developability of drug candidates within Pharmaceutical industry.

receiving values of a plurality of pharmacokinetic (PK) parameters (PPP) pertaining to one or more drug candidates ⟶ 202

preprocessing the value of plurality of PK parameters to obtain preprocessed values of the plurality of PK parameters, in accepted units ⟶ 204

assigning a lower bound to each of the PPP ⟶ 206

determining an upper bound for each of the PPP, the step of determining the upper bound for each of the PPP comprises: arranging and sorting each of the preprocessed values of the PPP in a pre-defined order to obtain a sorted list of values of the PPP; computing one or more percentiles values from the sorted list of values of the PK parameters; testing a usefulness of the one or more computed percentile values as one or more upper bounds; and determining a percentile value amongst the one or more percentile values as the upper bound ⟶ 208

identifying universal PK parameters' bounds comprising parameter names, the lower bound, the upper bound and the accepted units ⟶ 210

performing a global optimization technique on the plurality of pharmacokinetic (PK) parameters using the universal PK parameters' bounds and a plasma-concentration time (PCT) profile of the one or more drug candidates to obtain a globally optimized set of PK parameters for a given PK model ⟶ 212

performing a local optimization technique on the globally optimized set of PK parameters to obtain a locally optimized set of PK parameters, wherein the locally optimized set of PK parameters are used for assessing the developability of the one or more drug candidates ⟶ 214

**FIG. 2**

EP 4 510 138 A1

**Description**

CROSS-REFERENCE TO RELATED APPLICATIONS AND PRIORITY

The present application claims priority to Indian application no. 202321054496, filed on August 14, 2023.

TECHNICAL FIELD

The disclosure herein generally relates to estimation of Pharmacokinetic (PK) parameters, and, more particularly, to systems and methods for estimation of Pharmacokinetic (PK) parameters of drug candidates using universal PK parameters bounds.

BACKGROUND

Developability of a drug candidate is decided based on the Pharmacokinetic (PK) and Pharmacodynamic (PD) parameters of the drug candidate under investigation, usually estimated from Plasma Concentration Time (PCT) and response time profiles of the drug candidate, measured in several targeted animal/human in vivo studies. PK is the study of what the body does to a drug, i.e., its absorption, distribution, metabolism, and excretion profiles. On the other hand, PD defines what a drug does to the body.

The objective of these models is to estimate the PK and PD parameters which is an integral part of model-based drug development. The significance of accurately estimating these parameters lies in the fact that many of these parameters cannot be measured experimentally either in human or in animal. Further, the decision to take the compound/drug to the next level of drug development process depends heavily on the accuracy of the parameters' values. The accuracy of the optimized parameters obtained using available optimization methods such as Gauss-Newton, Nelder Mead etc., is dependent on appropriate selection of initial parameter values, which intern is critically dependent on the selected parameter bounds. These estimations may be an educated guess based on the structure of the drug candidate or can be estimated using non-compartmental analysis or curve striping, grid search etc. approaches. However, these approaches do not always guarantee good initial parameter values of all the PK parameters of interest. Further, the number of parameters and complexity of the model increase the execution time required for a computing method to estimate the parameters. Grid search (GS) method and nature inspired global optimization techniques such as genetic algorithm, artificial immune networks were proposed and used in the literature to calculate initial parameter values from parameter bounds. To take advantage of these methods, the user has to provide bounds of the parameters that are as close as possible to that of the initial parameters, and is critical for the estimation of good initial parameter values. Thus, selection of a good parameter bounds is an extremely important step in the estimation of PK parameters and any approach that does not require domain expertise of the user, will lead to easy adaptation in Pharmaceutical industry setup. So, there is a need to generate these bounds through some system/process and do away with user dependency for this crucial information. This would also help to make the parameter optimization process a user intervention free automatic process.

SUMMARY

Embodiments of the present disclosure present technological improvements as solutions to one or more of the above-mentioned technical problems recognized by the inventors in conventional PK parameter estimation systems.

For example, in one aspect, there is provided a processor implemented method for estimation of Pharmacokinetic (PK) parameters of drug candidates using universal PK parameters' bounds. The method comprises receiving, via one or more hardware processors, values of a plurality of pharmacokinetic (PK) parameters pertaining to drug candidates; preprocessing, via the one or more hardware processors, the values of the plurality of PK parameters to obtain preprocessed values of the plurality of PK parameters, in accepted units; assigning, via the one or more hardware processors, a lower bound (typically zero) to each of the plurality of PK parameters; determining, via the one or more hardware processors, an upper bound for each of the plurality of PK parameters, wherein the steps of determining the upper bound for each of the plurality of PK parameters comprises: arranging and sorting the preprocessed values of the plurality of PK parameters in a pre-defined order to obtain a sorted list of values of PK parameters; computing one or more percentiles for the sorted list of values of the PK parameters; testing usefulness of the one or more computed percentiles as one or more upper bounds; and determining a percentile value amongst the one or more percentile values as the upper bound; identifying, via the one or more hardware processors, one or more universal PK parameters' bounds comprising one or more parameter names, the lower bound, the upper bound, and the accepted units; performing, via the one or more hardware processors, a global optimization technique on the plurality of pharmacokinetic (PK) parameters using the one or more universal PK parameters' bounds and a plasma-concentration time (PCT) profile of the one or more drug candidates to obtain a globally optimized set of PK parameters for a given PK model; and performing, via the one or more hardware processors, a local optimization technique on the globally optimized set of PK parameters to obtain a locally optimized set of PK parameters, wherein the locally optimized set of PK parameters are used for assessing the developability of the one or more drug candidates.

In an embodiment, the globally optimized set of PK parameters is obtained by dynamically creating a combination of multiple bounds generated from the one or more universal PK parameters' bounds, using a logical operation technique on one or more universal PK parameters' bounds, the automatic logical operation technique comprises: performing one or more logical operations on the upper bound of the universal PK parameters' bounds of the plurality of PK parameters; and generating one or more upper bound combinations based on one or more values obtained after logical operations for each of the plurality of PK parameters to obtain the combination of multiple bounds comprising upper bound combinations and zero as lower bound for all of the combinations; and estimating PK parameters of the given PK model from the combination of multiple bounds using the global optimization technique, wherein the estimated PK parameters serve as the globally optimized set of PK parameters.

In an embodiment, a best set of PK parameters amongst the locally optimized set of PK parameters is obtained by performing the local optimization technique on each of the globally optimized set of PK parameters from the combination of multiple bounds to obtain a set of locally optimized set of PK parameters, wherein the best set of PK parameters amongst the locally optimized set of PK parameters is selected based on the associated weighted residual sum squares (WRSS) value.

In an embodiment, the method comprises sequentially performing the global optimization technique and the local optimization technique for the combination of multiple bounds generated from the universal PK parameters' bounds to obtain the locally optimized set of PK parameters; and selecting a best set of PK parameters based on a comparison of an associated residual sum squares (RSS) value of each of the locally optimized set of PK parameters and the pre-defined performance threshold of the RSS value.

In another aspect, there is provided a processor implemented system for estimation of Pharmacokinetic (PK) parameters of drug candidates based on universal PK parameters' bounds. The system comprises: a memory storing instructions; one or more communication interfaces; and one or more hardware processors coupled to the memory via the one or more communication interfaces, wherein the one or more hardware processors are configured by the instructions to: receive values of a plurality of pharmacokinetic (PK) parameters pertaining to drug candidates; preprocess the values of the plurality of PK parameters to obtain values of the plurality of PK parameters in accepted units; assign a lower bound to each of the plurality of PK parameters; determine, an upper bound for each of the plurality of PK parameters, wherein the step of determining the upper bound for each of the plurality of PK parameters comprises: arranging and sorting the preprocessed values of the plurality of PK parameters in a pre-defined order to obtain a sorted list of values of the PK parameters; computing one or more percentiles values for the sorted list of values of PK parameters to obtain one or more percentile values; testing usefulness of the one or more computed percentile values as the one or more upper bounds; and determining a percentile value amongst the one or more percentile values as the upper bound; identify one or more universal PK parameters' bounds comprising one or more parameter names, the lower bound, the upper bound and the accepted units; perform a global optimization technique on the plurality of pharmacokinetic (PK) parameters using the universal PK parameters' bounds and a plasma-concentration time (PCT) profile of the one or more drug candidates to obtain a globally optimized set of PK parameters for a given PK model; and perform a local optimization technique on the globally optimized set of PK parameters to obtain a locally optimized set of PK parameters, wherein the locally optimized set of PK parameters are used for assessing developability of the one or more drug candidates.

In an embodiment, the globally optimized set of PK parameters is obtained by dynamically creating a combination of multiple bounds generated from the one or more universal PK parameters' bounds, using a logical operation technique on one or more universal PK parameters' bounds, the automatic logical operation technique comprises: performing one or more logical operations on the upper bound of the universal PK parameters' bounds of the plurality of PK parameters; and generating one or more upper bound combinations based on one or more values obtained after logical operations for each of the plurality of PK parameters to obtain the combination of multiple bounds comprising upper bound combinations and zero as lower bound for all of the combinations; and estimating PK parameters of the given PK model from for the combination of multiple bounds using the global optimization technique, wherein the estimated PK parameters serve as the globally optimized set of PK parameters.

In an embodiment, a best set of PK parameters amongst the locally optimized set of PK parameters is obtained by performing the local optimization technique on each of the globally optimized set of PK parameters from the combination of multiple bounds to obtain a set of locally optimized set of PK parameters, wherein the best set of PK parameters amongst the locally optimized set of PK parameters is selected based on the associated weighted residual sum squares (WRSS) value.

In an embodiment, the one or more hardware processors are further configured by the instructions to sequentially perform the global optimization technique and the local optimization technique for the combination of multiple bounds generated from the one or more universal PK parameters' bounds to obtain the locally optimized set of PK parameters; and select a best set of PK parameters based on a comparison of an associated residual sum squares (RSS) value of each of the locally optimized set of PK parameters and the pre-defined performance threshold of the RSS value.

In yet another aspect, there are provided one or more non-transitory machine-readable information storage mediums comprising one or more instructions which when executed by one or more hardware processors cause estimation of Pharmacokinetic (PK) parameters of drug candidates based on universal PK parameters' bounds by receiving values of a plurality of pharmacokinetic (PK) parameters pertaining to drug candidates; preprocessing the values of the plurality of PK parameters to obtain preprocessed values of the plurality of PK parameters, in accepted units; assigning a lower bound to each of the plurality of PK parameters; determining an upper bound for each of the plurality of PK parameters, wherein the step of determining the upper bound for each of the plurality of PK parameters comprises: arranging and sorting the preprocessed values of the plurality of PK parameters in a pre-defined order to obtain a sorted list of values of the PK parameters; computing one or more percentile values for the sorted list of values of the PK parameters; testing usefulness of the one or more computed percentile values as one or more upper bounds; and determining a percentile value amongst the one or more percentile values as the upper bound; identifying one or more universal PK parameters' bounds comprising one or more parameter names, the lower bound, the upper bound and the accepted units; performing a global optimization technique on the plurality of pharmacokinetic (PK) parameters using the universal PK parameters' bounds and a plasma-concentration time (PCT) profile of the one or more drug candidates to obtain a globally optimized set of PK parameters for a given PK model; and performing a local optimization technique on the globally optimized set of PK parameters to obtain a locally optimized set of PK parameters, wherein the locally optimized set of PK parameters are used for assessing developability of the one or more drug candidates.

In an embodiment, the globally optimized set of PK parameters is obtained by dynamically creating a combination of multiple bounds generated from the one or more universal PK parameters' bounds, using a logical operation technique on one or more universal PK parameters' bounds, the automatic logical operation technique comprises: performing one or more logical operations on the upper bound of the universal PK parameters' bounds of the plurality of PK parameters; and generating one or more upper bound combinations based on one or more values obtained after logical operations for each of the plurality of PK parameters to obtain the combination of multiple bounds comprising upper bound combinations and zero as lower bound for all of the combinations; and estimating PK parameters of the given PK model from the combination of multiple bounds using the global optimization technique, wherein the estimated PK parameters serve as the globally optimized set of PK parameters.

In an embodiment, a best set of PK parameters amongst the locally optimized set of PK parameters is obtained by performing the local optimization technique on each of the globally optimized set of PK parameters of the given PK model from the combination of multiple bounds to obtain a set of locally optimized set of PK parameters, wherein the best set of PK parameters amongst the locally optimized set of PK parameters is selected based on the associated weighted residual sum squares (WRSS) value.

In an embodiment, the one or more instructions which when executed by one or more hardware processors further cause sequentially performing the global optimization technique and the local optimization technique for the combination of multiple bounds generated from the one or more universal PK parameters' bounds to obtain the locally optimized set of PK parameters; and selecting a best set of PK parameters based on a comparison of an associated residual sum squares (RSS) value of each of the locally optimized set of PK parameters and the pre-defined performance threshold of the RSS value.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system for estimation of Pharmacokinetic (PK) parameters of drug candidates using one or more universal PK parameters' bounds, in accordance with an embodiment of the present disclosure.
FIG. 2 depicts an exemplary flow chart illustrating a method for estimation of Pharmacokinetic (PK) parameters of drug candidates using the one or more universal PK parameters' bounds, using the system of FIG. 1, in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Estimating PK parameters is an integral part of model-based drug development. The significance of accurately estimating these parameters lies in the fact that many of these parameters cannot be measured experimentally either in human or in animal. Further, the decision to take the compound/drug to the next level of drug development process depends heavily on the accuracy of the parameters' values. The accuracy of the optimized parameter estimates obtained using available optimization methods such as Gauss-Newton, Nelder Mead, etc., is dependent on appropriate selection of initial parameter values. These estimations may be an educated guess based on the structure of the drug candidate or can be estimated using non-compartmental analysis or curve striping, grid search etc. approaches. However, these approaches do not always guarantee good initial estimates of all the PK parameters of interest.

All commercial and public tools, software or methods require at least one of the a) initial parameter values and b) parameter bounds for estimating optimal PK parameters. The success of these methods, defined in terms of convergence, optimality, speed, accuracy, memory consumption, resource utilization and others, depends on the quality of both the initial parameter values and the PK parameter bounds. However, computation of appropriate initial parameter values, by the user, that assist in convergence of the optimization method is not a straightforward task. Providing appropriate initial parameter values require comprehensive knowledge of pharmacokinetics, which may be scarce and is one of the bottlenecks for PK parameter estimation. Further, most of the PK optimization methods in commercial tools fail to obtain optimal parameters due to convergence issues or need greater execution times and memory to obtain optimal estimates. Hence, a good initial estimate of parameters for PK parameter optimization becomes a critical component for success of optimization process. Embodiments of the present disclosure provide systems and methods for PK parameter optimization process that ensure a good initial estimate is obtained for PK optimization process, addressing the bottlenecks discussed above.

Two step optimization process has been in practice in literature to address the dependency on initial parameter values. This process consists of two stages; in stage1, initial parameters (IP) are computed using an initial parameter estimation method, preferably a global optimization technique and user provided parameter bounds and in stage2, the IP estimated in stage1 is then optimized using another set of optimization methods, more specially named local optimization techniques. Some of the estimation methods used in stage1 are grid search, genetic algorithm, league championship algorithm etc. The drawback of the above mentioned two stage process is that user has to provide the parameter bounds of the model, from which the initial parameters are estimated, which subsequently, further optimized using local optimization techniques to get the final parameters. The overall success of the estimation of the final Parameters is dependent on i) appropriate parameter bounds ii) methods to estimate the initial PK parameters iii) local optimization of the initial parameters to get the final PK parameters. The present disclosure is designed to address aforementioned issue of the PK parameter estimation process which makes use of newly developed universal PK parameters' bounds (UPKPB), logical operations on universal bounds(e.g., by providing option to perform division and/or multiplication of UPKPB), performance threshold on residual sum of squares (RSS) and global optimization techniques such as modified wind driven optimization and local optimization technique such as Gauss Newton, Nelder Mead, etc. to produce optimal PK parameters. Here, the global optimization technique used is a modified version of publicly available wind driven optimization. The term 'Universal PK Parameters' Bounds (UPKPB)" refers to lower and upper bounds of the PK parameters that are used in the estimation of initial PK parameter using global optimization methods. The universal PK parameters' bounds can be used for any case study and thus, it is not case study specific. The lower bound value is typically zero and the upper bound, is determined using data mining of the parameters of marketed drugs, using a set of rules, described in detail in the disclosure. The parameters have clearly defined units that are accepted within research community. For example, in the case of the PK parameter, "volume of distribution", $V_d$, the accepted unit is L/Kg. The term "globally optimized parameters" or "globally optimized set of PK parameters" or "Initial PK parameters" are synonymous. It can be calculated from user defined parameters bounds or universal PK parameters' bounds with the use of global optimization methods. The term "locally optimized PK Parameters" or "locally optimized set of PK parameters" are synonymous and refers to the final or best PK parameters of a drug candidate and are estimated using local optimization techniques and initial PK parameters.

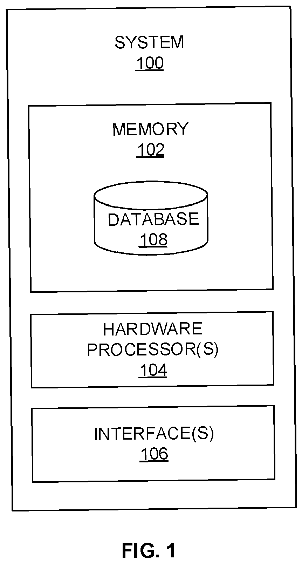

Referring now to the drawings, and more particularly to FIGS. 1 through 2, where similar reference characters denote corresponding features consistently throughout the figures, there are shown preferred embodiments and these embodiments are described in the context of the following exemplary system and/or method.

FIG. 1 depicts an exemplary system 100 for estimation of Pharmacokinetic (PK) parameters of drug candidates based on universal PK parameters' bounds, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information on a plurality of pharmacokinetic (PK) parameters pertaining to drug candidates, preprocessed value of PK parameters, and the like. The database 108 further comprises information pertaining to universal PK parameters' bounds comprising the lower bound and the upper bound, a globally optimized set of PK parameters for a given PK model, a locally optimized set of PK parameters, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

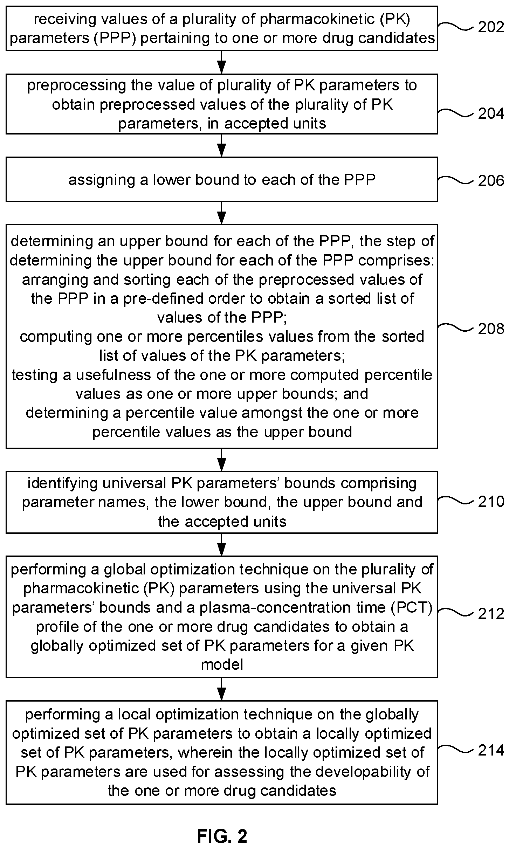

FIG. 2, with reference to FIG. 1, depicts an exemplary flow chart illustrating a method for estimation of Pharmacokinetic (PK) parameters of drug candidates based on universal PK parameters' bounds, using the system 100 of FIG. 1, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, and the flow diagram as depicted in FIG. 2. Although steps of the method of FIG. 2 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method of the present disclosure, the one or more hardware processors 104 receive values of a plurality of pharmacokinetic (PK) parameters pertaining to drug candidates (also referred to as compounds and interchangeably used herein). The above step 202 is better understood by way of following description:

The system 100 explored literature/publications to collect PK parameters of drug candidates. 'Obach, R. Scott, Franco Lombardo, and Nigel J. Waters. "Trend analysis of a database of intravenous pharmacokinetic parameters in humans for 670 drug compounds". Drug Metabolism and Disposition 36.7 (2008): 1385-1405' analyzed trend of pharmacokinetics parameters obtained after intravenous administration of 670 medicinal compounds. Five pharmacokinetic PK parameters-volume of distribution at steady state (VDss), Clearance (Cl), Mean Residence Time (MRT), and terminal phase half-life ($t_{1/2}$), have been used for the analysis by the system 100, along with plasma protein binding data. To examine more thoroughly and collect other PK parameters, 342 publications corresponding to the 331 drug candidates mentioned in the above publication were downloaded and analyzed. The literature/publications include but are not limited to 1) "BOWER, SUSANNE, and C. J. Hull. "Comparative pharmacokinetics of fentanyl and alfentanil." British Journal of Anaesthesia 54.8 (1982): 871-877" 2) "Ripa, S., L. Ferrante, and M. Prenna. "A linear model for the pharmacokinetics of azithromycin in healthy volunteers. " Chemotherapy 42.6 (1996): 402-409" 3) "Barnett, Gene, Richard Hawks, and Richard Resnick. "Cocaine pharmacokinetics in humans. " Journal of ethnopharmacology 3.2-3 (1981): 353-366. ". Total number of plasma concentration-time (PCT) profiles available in these 342 publications is 453 and compartment analysis has been used to model the PCT profiles. The present disclosure and its system and method aimed at aggregating highest values of individual pharmacokinetic (PK) parameters from the publications. Below are details:

Further, a set of rules have been defined by the system 100 and the method described herein to carry out the parameter collection process from the downloaded publications. Different possible scenarios of PK parameters study/analysis (such as patient and/or volunteers as subjects upon which the experiments are carried out, use of single or multiple PK models, different doses) and representation of PK parameters (such as individual values of each parameter for each subject, maximum and minimum value of the parameters over all the subjects, mean and standard deviation of the parameters) have been considered and rules have been defined to take care of these scenarios. Below are various rules illustrated by way of examples:

Rule1: If in a publication, a parameter value is provided for all the subjects, then maximum value over all the subjects is considered as the selected parameter value for that publication. Below Table1 is a representation of this rule. More specifically, Table 1 illustrates representation of estimated PK parameter values of Alfentanil on 7 subjects and selected parameter value using Rule 1. In the publication *"BOWER, SUSANNE, and C. J. Hull. "Comparative pharmacokinetics of fentanyl and alfentanil." British Journal of Anaesthesia 54.8 (1982): 871-877"*, 7 different estimated values (8.94, 11.1, 11, 8.26, 13.04, 9.15, 15.17) of parameter V1 have been provided corresponding to 7 different subjects used for the study of alfentanil. The value of V1 for subject 7 is maximum (15.17) and it is considered as the selected value of V1 for this publication.

Table 1

| Compound | ROA & Dose | Parameter Name | unit | Subject | | | | | | | Selected Parameter Value |
|---|---|---|---|---|---|---|---|---|---|---|---|
| | | | | 1 | 2 | 3 | 4 | 5 | 6 | 7 | |
| Alfentanil | IV Bolus – 161.74 μg | V1 | L | 8.94 | 11.1 | 11 | 8.26 | 13.04 | 9.15 | 15.17 | 15.17 |
| | | V2 | L | 16.22 | 24.18 | 12.25 | 18.26 | 10.17 | 9.34 | 23.52 | 24.18 |
| | | Vss | L | 25.16 | 35.28 | 23.25 | 26.52 | 23.21 | 18.49 | 38.69 | 38.69 |
| | | K12 | 1/hr | 3.24 | 5.39 | 2.21 | 5.12 | 0.91 | 3.76 | 2.47 | 5.39 |
| | | K21 | 1/hr | 1.79 | 2.47 | 1.98 | 2.31 | 1.17 | 3.68 | 1.59 | 3.68 |
| | | K10 | 1/hr | 1.71 | 2.11 | 1.11 | 1.27 | 0.61 | 1.36 | 1.2 | 2.11 |
| | | CL | L/hr | 15.26 | 23.38 | 12.29 | 10.49 | 7.91 | 12.41 | 18.24 | 23.38 |
| | | T1/2beta | hr | 1.42 | 1.25 | 1.52 | 1.97 | 2.34 | 1.14 | 1.76 | 2.34 |

Rule 2: If in a publication, parameter range i.e., minimum, and maximum value of the parameters over all the subject is provided, then maximum value is considered as the selected parameter value for that publication. Below Table 2 is a representation of this case sourced from the publication *"Ripa, S., L. Ferrante, and M. Prenna. "A linear model for the pharmacokinetics of azithromycin in healthy volunteers." Chemotherapy 42.6 (1996): 402-409"*. More specifically, Table 2 illustrates mean, minimum and maximum values of the PK parameters for subjects considered for the study of Azithromycin and selected parameter value using Rule 2. For parameter K12, maximum estimated value is 0.18 and it is captured as the selected value for K12.

Table 2

| Compo und Name | ROA & Dose | Parameter | | Parameter Value | | | Selected Paramet er Value |
|---|---|---|---|---|---|---|---|
| | | Nam e | Unit | Mean Value | Minim um | Maximu m | |
| Azithro mycin | Oral - 500m g | K12 | 1/hr | 0.12 | 0.07 | 0.18 | 0.18 |
| | | K21 | 1/hr | 0.04 | 0.03 | 0.06 | 0.06 |
| | | K10 | 1/hr | 0.08 | 0.05 | 0.12 | 0.12 |
| | | V1 | L | 586.83 | 341.94 | 1025.35 | 1025.35 |
| | | V2 | L | 1779.1 2 | 798.71 | 3193.05 | 3193.05 |
| | | Ke | 1/hr | 0.02 | 0.02 | 0.02 | 0.02 |
| | | Vd beta | L | 3219.5 1 | 1593.4 1 | 5475.92 | 5475.92 |
| | | Vss | L | 2347.9 5 | 1018.7 8 | 4218.4 | 4218.4 |
| | | T1/2 beta | hr | 55.46 | 38.09 | 76.17 | 76.17 |
| | | AUC | μg.hr/ml | 4.86 | 3.5 | 6.38 | 6.38 |
| | | CL | ml/min | 664.84 | 110.91 | 280.35 | 280.35 |

Rule 3: If in a publication, mean and standard deviation (SD) of the PK parameter are provided, then it is not possible to find out the maximum value estimated for a single subject. So, for this scenario, (mean + SD) of the parameter is considered as the selected parameter value. Below Table 3 is a representation of this rule explained with the study presented in "Stagni, Grazia, Patrick J. Davis, and Thomas M. Ludden. "Human pharmacokinetics of betaxololenantiomers." Journal of pharmaceutical sciences 80.4 (1991): 321-324*"*. More specifically, Table 3 illustrates representation of estimated parameters in the form of mean and SD for Betaxolol and selected parameter value using Rule 3. For PK parameter Vz, the mean and standard deviation are 5.07 and 0.74 respectively. So, 5.07+0.74 = 5.81 is considered as selected value of Vz from this publication.

Table 3

| Compound name | ROA & Dose | Parameter | | | | |
|---|---|---|---|---|---|---|
| | | Name | units | Mean Value | SD | Selected Parameter Value |
| Betaxolol | IV infusion - 10 mg | Vz | L/Kg | 5.07 | 0.74 | 5.81 |
| | | CL | L/hr | 15.1 | 2.9 | 18 |
| | | AUC | μg.hr/L | 610 | 122 | 732 |
| | | Lambda Z | 1/hr | 0.0415 | 0.0050 | 0.0465 |

Rule 4: If in a publication, multiple PK models are used, then the highest parameter value across the models is considered as the selected parameter value for that publication. An example of this rule is sourced from "Grimaldi, R., et al. "Pharmacokinetic and pharmacodynamic studies following the intravenous and oral administration of the antiparkinsonian drug biperiden to normal subjects. " European journal of clinical pharmacology 29.6 (1986): 735-737*"*, is presented in Table 4. More specifically, Table 4 illustrates estimated PK parameters value of Biperiden for oral and infusion dose and selected parameter value using Rule 4. In this publication, two different PK models - 2 compartments oral and 2 compartments IV Infusion have been analyzed. The values of parameter Alpha are 1.11 and 0.6 for 2 compartments oral and 2 compartments IV Infusion respectively. So, the maximum value of Alpha is 1.11 and it is collected as the selected value of Alpha for this publication.

Table 4

| Compound Name | ROA | Model | Parameter | | | | |
|---|---|---|---|---|---|---|---|
| | | | Name | Units | Value | SD | Selected Parameter Value |
| Biperiden | Oral - 4mg | 2 compartment | Lag time | hr | 0.46 | NA | 0.46 |
| | | | Ka | 1/hr | 2.18 | NA | 2.18 |
| | | | Alpha | 1/hr | 1.11 | NA | 1.11 |
| | | | Beta | 1/hr | 0.030-0.050 | NA | 0.05 |
| | IV Infusion, 4 mg for 5 minutes | 2 compartment | Alpha | 1/hr | 0.53 | 0.07 | 0.6 |
| | | | T1/2 Alpha | hr | 1.5 | 0.2 | 1.7 |
| | | | Beta | 1/hr | 0.03 | 0.01 | 0.04 |
| | | | T1/2 beta | hr | 24.3 | 3.9 | 28.2 |
| | | | K12 | 1/hr | 0.1910 | 0.0027 | 0.19 |
| | | | K21 | 1/hr | 0.06 | 0.01 | 0.07 |
| | | | Kel | 1/hr | 0.31 | 0.05 | 0.36 |
| | | | AUC | ng.h/m l | 62.8 | 2.6 | 65.4 |
| | | | V1 | L/kg | 2.6 | 0.4 | 3 |
| | | | Varea | L/kg | 11.5 | 4.1 | 15.6 |
| | | | CL | ml/min /kg | 24 | 0.8 | 24.8 |

Rule 5: If in a publication, patients and volunteers have been used as subjects and patients' and volunteers' parameter values are provided separately, then maximum value (Rule 1)/ maximum of range (Rule 2)/ maximum of (mean + SD) (Rule 3) across all the subjects (patients and volunteers) of each parameter is considered as the selected parameter value for that publication.

Rule 6: If in a publication, multiple compartmental models and multiple estimation methods are used, then maximum value/maximum of range/ mean + SD across all the models and methods of each PK parameter is considered as the selected PK parameter value for that publication.

Rule 7: If in a publication, multiple analysis has been carried out using multiple doses, then the maximum value provided over all the doses for each parameter is considered as the selected parameter value for that publication. The example for this rule is sourced from "Barnett, Gene, Richard Hawks, and Richard Resnick. "Cocaine pharmacokinetics in humans. " Journal of ethnopharmacology 3.2-3 (1981): 353-366." and presented in Table 5. More specifically, Table 5 illustrates a representation of estimated parameter values of Cocaine for two different doses of 100mg and 200mg and one compartment and two compartment models. In this publication, the value of parameter Alpha over all the subjects and doses are 0.0135, 0.0172, 0.0147, 0.0532, 0.00879. The maximum value among these is 0.0532 and it is the selected value of Alpha for this publication. Parameter A and B which are the coefficient of bi-exponential model follow a different rule which is defined subsequently and selected value for them in Table 5 is mentioned as not applicable (NA).

Table 5

| Compoun d name | ROA & Dos | | | Dose = 100 mg | | | Dose = 200 mg | | |
| --- | --- | --- | --- | --- | --- | --- | --- | --- | --- |
| | | | | Parameter value | | | Parameter value | | Selec ted Value |
| | | Para meter | Unit | Sub-W | Sub-X | Sub-Y | Sub-W | Sub-Z | |
| Cocai n | IV bolus , dose 100, 200 mg | A | ng/ml | 860 | 558 | 1139 | 2482 | 1432 | NA |
| | | Alph a | 1/min | 0.013 5 | 0.017 2 | 0.014 7 | 0.053 2 | 0.008 79 | 0.053 2 |
| | | B | ng/ml | | | | 1997 | | NA |
| | | Beta | 1/min | | | | 0.008 42 | | 0.008 42 |

Rule 8: The PK parameters (A, B and C) corresponding to the coefficients of sum of exponential models are dose dependent parameters and their value can be computed roughly through method of residual (curve stripping). The values of A, B, C computed through this method are supposed to be less than Cmax (maximum value of concentration). Additionally, based on experience of PK data analysis, it has been observed that though the values of A, B and C vary over wide range, the values can be restricted by 2*Cmax for A and Cmax for B and C. So, Cmax based bound is used for A, B and C and they are omitted from the subsequent process of bound selection. To maintain uniformity, for all of A, B and C, 2*Cmax is considered as upper bound of them in universal parameters' bounds. The example for this rule is sourced from "Barnett, Gene, Richard Hawks, and Richard Resnick. "Cocaine pharmacokinetics in humans." Journal of ethnopharmacology 3.2-3 (1981): 353-366" and presented in Table 6. More specifically, Table 6 illustrates representation of estimated PK parameter values and selected upper bound of cocaine modeled by a bi-exponential model. The estimated values of A and B are 2482 and 1997 ng/ml respectively. The upper bound proposed through this rule is 7736 ng/ml for both A and B which is greater than the estimated values of both A and B.

Table 6

| Compou nd Name | ROA & Dose | Parameters | | | |
| --- | --- | --- | --- | --- | --- |
| | | Name | Unit | Value | Selected value/ Upper Bound |
| Cocaine | IVBol us - 200m 9 | A | ng/ml | 2482 | 7736 (Cmax = 3868) |
| | | Alpha | 1/min | 0.05 | 43.05 |
| | | B | ng/ml | 1997 | 7736 (Cmax = 3868) |
| | | Beta | 1/min | 0.08 | 9.01 |

Rule 9: Body weight of the subjects are required for unit conversion, which is explained later, for some parameters such as V1, V2, V3, Vss etc. Instead of using a fixed predefined value for all the compounds/publications, the maximum weight/ maximum of weight range/ maximum of weight (mean + SD) provided in the corresponding publication is used as body weight of the subject. The example of this rule is taken from "Cundy, Kenneth C., et al. "Clinical pharmacokinetics of adefovir in human immunodeficiency virus type 1-infected patients. " Antimicrobial agents and chemotherapy 39.11 (1995): 2401-2405"and presented in Table 7. More specifically, Table 7 illustrates representation of body weights of the subjects considered for the study of Adefovir and selection of body weight for unit conversion of parameters. Here, a mean (72.8) and SD (12) of body weights of the subjects have been provided. The (mean + SD) = 72.8+12 = 84.8 kg would be used as body weight of the subjects during unit conversion for this publication.

Table 7

| Compound Name | ROA & Dose | Name | Units | Mean Value | SD | selected weight for Unit Conversion |
| --- | --- | --- | --- | --- | --- | --- |
| Adefovir | IVinf - 3mg/kg | Weight | kg | 72.8 | 12 | 84.8 |

Based on the abovementioned rules, PK parameter values are collected for individual parameter from the publications.

Referring to steps of FIG. 2, at step 204 of the method of the present disclosure, the one or more hardware processors 104 preprocess the values of the plurality of PK parameters to obtain the preprocessed values of the plurality of PK parameters in accepted units. In other words, the collected values of the PK parameter (or plurality of PK parameters) are then converted for obtaining parameter values in accepted units by performing unit conversion whenever necessary. The accepted units of the parameters for time, volume, body weight and dose are hr, L, kg, ug respectively. Below Table 8 is a representation of the unit conversion of the PK parameters collected from "Stapleton, Stacie L., et al. "Plasma and cerebrospinal fluid pharmacokinetics of pemetrexed after intravenous administration in non-human primates. " Cancer chemotherapy and pharmacology 59.4 (2007): 461-466.". More specifically, Table 8 illustrates representation of unit conversion of parameters in the study of Trimetrexate. In this publication, unit of K10 is 1/min and to convert this to accepted unit of 1/hr, the selected value of K10 (which is 0.0516) has to be multiplied by 60. So, the unit converted selected value of K10 is 3.096 with accepted unit 1/hr.

Table 8

| Compou nd name | ROA and Dose | Parameter Name | Unit | Selected Paramet er Value | UPKPB Unit | Convert ed value |
|---|---|---|---|---|---|---|
| Trimetr exate | IV infusi on of 20mg/ kg for 10 minut es | K10 | 1/min | 0.0516 | 1/hr | 3.096 |
| | | K12 | 1/min | 0.0145 | 1/hr | 0.87 |
| | | K21 | 1/min | 0.0216 | 1/hr | 1.296 |
| | | K13 | 1/min | 0.00012 5 | 1/hr | 0.0075 |
| | | K31 | 1/min | 0.00155 | 1/hr | 0.093 |
| | | V1 | L/kg | 0.15 | L/kg | 0.15 |
| | | V2 | L/kg | 0.079 | L/kg | 0.079 |
| | | cl | ml/min | 4.2 | L/hr | 0.252 |
| | | T1/2 alpha | min | 18.3 | hr | 0.305 |
| | | T1/2 beta | min | 85.9 | hr | 1.43166 |

At step 206 of the method of the present disclosure, the one or more hardware processors 104 assign a lower bound (typically zero) to each of the plurality of PK parameters. The lower bound of each of the plurality of PK parameters is considered as zero, in accordance with an embodiment of the present disclosure.

At step 208 of the method of the present disclosure, the one or more hardware processors 104 determine, an upper bound for each of the plurality of PK parameters. The step of determining the upper bound for each of the plurality of PK parameters comprises arranging and sorting the preprocessed values of each of the plurality of PK parameters in a predefined order (e.g., say ascending order) to obtain a sorted list of values of the plurality of PK parameters. Below Table 9 illustrates all the selected parameter values (let the number of such values be N) of a PK parameter, CL, that are arranged and sorted in the ascending order.

Table 9

| Seria l No | CL (L/hr) | Serial No | CL (L/hr) | Serial No | CL (L/hr) | Serial No | CL (L/hr) | Serial No | CL (L/hr) |
|---|---|---|---|---|---|---|---|---|---|
| 1 | 0.095 | 51 | 3.75 | 101 | 9.52 | 151 | 25.5 | 201 | 62.25 6 |
| 2 | 0.105 | 52 | 4.14 | 102 | 9.61 | 152 | 25.74 1 | 202 | 62.69 3 |
| 3 | 0.155 | 53 | 4.319 | 103 | 10.09 8 | 153 | 25.9 | 203 | 63.40 7 |
| 4 | 0.161 | 54 | 4.41 | 104 | 10.09 8 | 154 | 26.64 | 204 | 64.24 7 |
| 5 | 0.163 | 55 | 4.5 | 105 | 10.5 | 155 | 27.1 | 205 | 64.4 |
| 6 | 0.167 | 56 | 4.535 | 106 | 10.5 | 156 | 27.2 | 206 | 64.4 |
| 7 | 0.17 | 57 | 4.697 | 107 | 10.62 4 | 157 | 27.29 5 | 207 | 64.4 |
| 8 | 0.252 | 58 | 4.7 | 108 | 10.67 8 | 158 | 27.95 4 | 208 | 65.12 3 |
| 9 | 0.27 | 59 | 4.721 | 109 | 11.5 | 159 | 28.42 | 209 | 65.68 8 |
| 10 | 0.288 | 60 | 4.806 | 110 | 11.7 | 160 | 28.85 4 | 210 | 66 |

(continued)

| Seria l No | CL (L/hr) | Serial No | CL (L/hr) | Serial No | CL (L/hr) | Serial No | CL (L/hr) | Serial No | CL (L/hr) |
|---|---|---|---|---|---|---|---|---|---|
| 11 | 0.291 | 61 | 4.919 | 111 | 12.41 8 | 161 | 28.97 | 211 | 69.04 6 |
| 12 | 0.296 | 62 | 5.1 | 112 | 13.19 1 | 162 | 29.38 2 | 212 | 69.88 6 |
| 13 | 0.302 | 63 | 5.117 | 113 | 13.3 | 163 | 29.39 4 | 213 | 72 |
| 14 | 0.37 | 64 | 5.14 | 114 | 13.74 9 | 164 | 31.5 | 214 | 72.1 |
| 15 | 0.384 | 65 | 5.165 | 115 | 13.92 | 165 | 32.69 | 215 | 72.8 |
| 16 | 0.42 | 66 | 5.34 | 116 | 14.9 | 166 | 34.43 3 | 216 | 75.39 |
| 17 | 0.42 | 67 | 5.35 | 117 | 15.4 | 167 | 35.10 5 | 217 | 75.6 |
| 18 | 0.424 | 68 | 5.375 | 118 | 15.70 5 | 168 | 35.39 3 | 218 | 77 |
| 19 | 0.427 | 69 | 5.677 | 119 | 15.88 5 | 169 | 37.49 9 | 219 | 78.65 |
| 20 | 0.456 | 70 | 5.725 | 120 | 16.1 | 170 | 38.27 2 | 220 | 78.86 |
| 21 | 0.58 | 71 | 5.88 | 121 | 16.2 | 171 | 38.84 2 | 221 | 80.5 |
| 22 | 0.726 | 72 | 5.9 | 122 | 16.5 | 172 | 38.91 | 222 | 81.5 |
| 23 | 0.752 | 73 | 5.981 | 123 | 16.81 8 | 173 | 39.9 | 223 | 83.14 |
| 24 | 0.8 | 74 | 5.999 | 124 | 17.05 8 | 174 | 42 | 224 | 83.3 |
| 25 | 0.822 | 75 | 6.047 | 125 | 17.1 | 175 | 42.59 1 | 225 | 84 |
| 26 | 1.19 | 76 | 6.119 | 126 | 17.5 | 176 | 43.8 | 226 | 84 |
| 27 | 1.26 | 77 | 6.299 | 127 | 18.05 6 | 177 | 44 | 227 | 84.82 3 |
| 28 | 1.296 | 78 | 6.3 | 128 | 19.04 | 178 | 46.3 | 228 | 87 |
| 29 | 1.309 | 79 | 6.473 | 129 | 19.14 | 179 | 47.72 | 229 | 88.9 |
| 30 | 1.368 | 80 | 6.593 | 130 | 19.74 | 180 | 48 | 230 | 90 |
| 31 | 1.47 | 81 | 6.68 | 131 | 19.74 | 181 | 48.4 | 231 | 91.7 |
| 32 | 1.529 | 82 | 6.77 | 132 | 19.74 | 182 | 49.55 | 232 | 94.2 |
| 33 | 1.609 | 83 | 6.81 | 133 | 19.81 | 183 | 50.22 2 | 233 | 95.9 |
| 34 | 1.747 | 84 | 6.839 | 134 | 19.85 6 | 184 | 50.45 | 234 | 96.34 8 |
| 35 | 1.91 | 85 | 6.98 | 135 | 20.07 2 | 185 | 51.89 | 235 | 99.4 |
| 36 | 2.1 | 86 | 6.99 | 136 | 20.37 | 186 | 52.7 | 236 | 101 |
| 37 | 2.206 | 87 | 7 | 137 | 20.48 | 187 | 53.2 | 237 | 104.1 3 |
| 38 | 2.3 | 88 | 7.09 | 138 | 20.49 2 | 188 | 53.2 | 238 | 104.7 6 |
| 39 | 2.37 | 89 | 7.47 | 139 | 20.49 2 | 189 | 53.21 | 239 | 106.4 |
| 40 | 2.388 | 90 | 7.475 | 140 | 21.7 | 190 | 53.52 | 240 | 107.0 7 |
| 41 | 2.39 | 91 | 7.7 | 141 | 22.3 | 191 | 53.92 9 | 241 | 109.1 7 |
| 42 | 2.66 | 92 | 7.87 | 142 | 22.67 5 | 192 | 54.16 9 | 242 | 110.8 5 |
| 43 | 2.68 | 93 | 8.614 | 143 | 22.97 | 193 | 54.58 9 | 243 | 113 |
| 44 | 2.88 | 94 | 8.84 | 144 | 23.1 | 194 | 56 | 244 | 116.7 3 |
| 45 | 3.011 | 95 | 8.97 | 145 | 23.51 5 | 195 | 56.68 9 | 245 | 116.9 |
| 46 | 3.059 | 96 | 8.998 | 146 | 23.52 | 196 | 57.34 9 | 246 | 130.2 9 |
| 47 | 3.401 | 97 | 9.04 | 147 | 24.01 9 | 197 | 59.85 | 247 | 148.2 3 |
| 48 | 3.479 | 98 | 9.18 | 148 | 24.6 | 198 | 60.02 1 | 248 | 149.0 7 |
| 49 | 3.724 | 99 | 9.364 | 149 | 24.61 | 199 | 61.47 6 | 249 | 150.7 9 |

(continued)

| Seria l No | CL (L/hr) | Serial No | CL (L/hr) | Serial No | CL (L/hr) | Serial No | CL (L/hr) | Serial No | CL (L/hr) |
|---|---|---|---|---|---|---|---|---|---|
| 50 | 3.746 | 100 | 9.38 | 150 | 25.2 | 200 | 61.6 | | |

Further, one or more percentile values for the sorted list of values of the plurality of PK parameters are computed. The usefulness of the one or more percentile values as upper bounds is tested and analyzed using the PCT profiles collected from the publications. One percentile value amongst the one or more percentile values is determined as the upper bound. The above steps of percentile value computation and upper bound determination are better understood by way of following description:

The aim of the system 100 and the method described herein is to find out the values of 50th, 75th, 90th and 98th percentile from the sorted list of PK parameters. Suppose a PK parameter has N values, then the required percentile values would be the N*50%, N*75%, N*90% and N*98% th values in the sorted list respectively. For the PK parameter CL, 249 representative values are obtained as presented in Table 9. The 50th percentile value of CL is 249*50/100 = 124.5= 124th (approximately) value in the ordered list of parameter value of CL which is 17.058 and 50% of the parameter value of CL is less than or equal to 17.058. Similarly, 75th percentile value is 249*75/100 = 186.75 = 186th value in the ordered list of parameter value which is 52.7 and 75% of the parameter value is less than or equal to 52.7. Similar calculation yields 83.3 and 116.74 as the 90th and 98th percentile values respectively for CL.

For further analysis, the 50th, 75th, 90th and 98th percentile values were then considered as options for upper bounds for universal parameters' bound and tested for their usefulness. Analyzing the optimized parameter values achieved using different bound options for the compartmental analysis of the plasma concentration-time (PCT) data collected from the abovementioned 342 publications, 90th percentile is found to be more realistic and robust as upper bound of the parameters. So, the list of PK parameters name, corresponding accepted unit, lower bound and the 90th percentile (upper bound) is proposed as one or more universal PK parameters' bounds (UPKPB). Accordingly, at step 210 of the method of the present disclosure, the one or more hardware processors 104 identify one or more universal PK parameters' bounds comprising one or more parameters names, the accepted units, the lower bound and the upper bound as mentioned above. Below Table 10 illustrates universal PK parameters' bounds for some of the PK parameters.

Table 10

| Parameter Name(s) | Accepted unit | Lower Bound | Upper Bound |
|---|---|---|---|
| CL | L/hr | 0 | 83.3 |
| V1 | L/Kg | 0 | 4.25 |
| Alpha | 1/hr | 0 | 30.18 |
| Beta | 1/hr | 0 | 1.466 |

At step 212 of the method of the present disclosure, the one or more hardware processors 104 perform a global optimization technique on the plurality of pharmacokinetic (PK) parameters using the one or more universal PK parameters' bounds and a plasma-concentration time (PCT) profile of the one or more drug candidates to obtain a globally optimized set of PK parameters for a given PK model. In an embodiment, global optimization technique is a modified wind driven optimization technique.

In the present disclosure, the global optimization method minimizes weighted residual sum squares (WRSS) to obtain globally optimized set of PK parameters. The WRSS is computed using the following formula.

$$WRSS = \sum_{i=1}^{n} w_i (C_i^{Obs} - C_i^{Pred})^2$$

Where 'n' is the number of observations in PCT profile ($C^{Obs}$, $t$), $C_i^{Obs}$ is the i-th value of the concentration, $C_i^{Pred}$ is i-th predicted value of the concentration which is computed during global optimization and $w_i$ is the weight of each of the observations. The different weighting options usually used are uniform, $1/C_i^{Obs}$, $1/(C_i^{Obs} * C_i^{Obs})$, $1/C_i^{Pred}$, $1/(C_i^{Pred} * C_i^{Pred})$ etc.

Below is an example of PCT profile, using which the globally optimized set of PK parameters are obtained for the given PK model.

The PCT Profile of a subject is collected from "Barnett, Gene, Richard Hawks, and Richard Resnick. "Cocaine pharmacokinetics in humans." Journal of ethnopharmacology 3.2-3 (1981): 353-366." and is illustrated in Table 11. The subject whose weight was 68 kg, was administered 200 mg of cocaine intravenously. The weighting option used for this profile is $1/(C_i^{Obs} * C_i^{Obs})$.

Table 11

| Time(hr) | Conc (μg/ml) |
|---|---|
| 0.08333333 | 3.868 |
| 0.33333333 | 2.476 |
| 0.65 | 2.031 |
| 1 | 1.222 |
| 2.13333333 | 0.696 |
| 3.2 | 0.453 |
| 4.16666667 | 0.219 |
| 5.15 | 0.145 |
| 6.15 | 0.094 |

A two compartment PK Model is used to estimate the parameters. The model is defined as C(t) = A*Exp(-Alpha*t) + B*Exp(-Beta*t) where A, B, alpha and beta are the four parameters whose value have to be estimated.

At the start of the parameter optimization process of the PK model, a query is made to universal PK parameters' bounds using parameter names of the PK model as the key. The UPKPB then returns the accepted unit, lower bound and upper bound of the parameters. If the accepted units in UPKPB and the units of a case study are different, the values of the upper bounds have to be converted according to the unit of the current study which then would be used for further computation and the same is presented in Table 12.

Table 12

| Parameter | Unit | Lower Bound | Upper Bound |
|---|---|---|---|
| A | μg/ml | 0 | 7.736 (2*Cmax) |
| B | μg/ml | 0 | 7.736 (2*Cmax) |
| Alpha | 1/hr | 0 | 30.18 |
| Beta | 1/hr | 0 | 1.466 |

The globally optimized value of the PK parameters is obtained using modified wind driven optimization (MWDO) technique. The MWDO technique uses PCT profile, dosing information, PK model, UPKPB of the parameters and weighting option as input and minimizes WRSS value to compute the globally optimized value as presented in Table 13. The minimized WRSS value is 0.10702. This globally optimized value of the PK parameters is considered as initial parameters and further optimized using local optimization methods in the subsequent step.

Table 13

| Parameter | Unit | Globally optimized value (initial parameter value) |
|---|---|---|
| A | μg/ml | 3.34016 |
| B | μg/ml | 2.49483 |
| Alpha | 1/hr | 9.48631 |
| Beta | 1/hr | 0.55344 |

At step 214 of the method of the present disclosure, the one or more hardware processors 104 perform local optimization technique on the globally optimized set of PK parameters, also known as initial PK parameters, to obtain a locally optimized set of PK parameters. The locally optimized set of PK parameters are used for assessing developability of the one or more drug candidates within pharmaceutical industry. The local optimization technique as implemented by the system 100 and the method is known as Nelder Mead optimization technique in the art. Other methods such as Gauss Newton, and the like may also be implemented herein. Here, Nelder Mead method locally optimized the globally optimized set of PK parameters provided in Table 13 by minimizing WRSS. The locally optimized set of PK parameters, also known as best parameters, is presented in Table 14 along with the published value. The WRSS for estimated best parameters and for published parameters are 0.05507 and 0.05507 respectively and thus, the current invention provides an industrially acceptable solution, for real world applications.

Table 14

| Parameter | Unit | Locally optimized value (Best parameter value) | Published Parameter Value |
|-----------|------|-----------------------------------------------|---------------------------|
| A | μg/ml | 2.46383 | 2.482 |
| B | μg/ml | 1.99643 | 1.997 |
| Alpha | 1/hr | 3.00824 | 3.192 |
| Beta | 1/hr | 0.50503 | 0.505 |

The method of the present disclosure dynamically generates the combinations of multiple bounds from the one or more universal PK parameters' bounds, using a logical operation technique on one or more universal PK parameters' bounds. The logical operation technique comprises performing one or more logical operations (e.g., division, multiplication, and the like) on the upper bound of the plurality of PK parameters and generating one or more upper bound combinations based on one or more values obtained after logical operations on each of the plurality of PK parameters. The above step of logical operation technique can be better understood by way of following description:

Generation of a set of parameter bounds dynamically from the universal PK parameters' bounds can be done in following way. The upper bounds of all the parameters of a PK model are divided by 1, d, $d^2$, $d^3$, ...$d^h$, where d and h are user-defined values and each of the resulting values is considered as a representative value of the upper bounds. The upper bound combinations are generated by considering all different values of the upper bounds of each parameter. The lower bounds remain same as fetched from UPKPB. Suppose there are two parameters in a PK model; A and B and the upper bounds accessed from UPKPB are a2 and b2 respectively. Also assume that the user defined values of both d and h is 2. Then the upper bound of each parameter is divided by 1, 2, and 4 and there are total (no of upper bound values)$^{number\ of\ parameters}$ = $3^2$ = 9 upper bound combinations and each of them is used as parameter upper bound along with the lower bound. All the combinations of parameter bounds are provided in Table 15.

Table 15

| Parameter | Lower Bound | Upper Bound | Values of Upper Bound |
|-----------|-------------|-------------|-----------------------|
| A | 0 | a2 | a2, a2/2, a2/4 |
| B | 0 | b2 | b2, b2/2, b2/4 |
| Parameter Bound combination [(LB$_A$, LB$_B$); (UB$_A$, UB$_B$)] | [(0,0);(a2, b2)], [(0,0); (a2, b2/2)], [(0,0); (a2, b2/4)], [(0,0); (a2/2, b2)], [(0,0); (a2/2, b2/2)], [(0,0); (a2/2, b2/4)], [(0,0); (a2/4, b2)], [(0,0); (a2/4, b2/2)], [(0,0); (a2/4, b2/4)] | | |

Similarly, bound expansion can be performed by multiplying the upper bounds of all the parameters by 1, d, $d^2$, $d^3$, ...$d^h$, where d and h are user defined values. Then the upper bound combinations are generated by considering all different values of the upper bounds of each parameter as presented in Table 15. The lower bounds remain same as fetched from UPKPB. Above-described bound division and multiplication can be used individually or together.

Below is an example of PCT profile, using which the globally optimized set of PK parameters are obtained for the given PK model using combination of multiple bounds.

PCT Profile of a subject is collected from "Van Hamme, Michael J., M. M. Ghoneim, and John J. Ambre. "Pharmacokinetics of etomidate, a new intravenous anesthetic." Anesthesiology 49.4 (1978): 274-277" and is presented in Table 16. The subject whose weight was 60.9 kg, was administered 18.3 mg of etomidate intravenously. The uniform weighting option is used for both global and local optimization.

Table 16

| Time (hr) | Conc (μg/ml) |
|-----------|--------------|
| 0.067 | 0.26 |
| 0.133 | 0.18 |

(continued)

| Time (hr) | Conc (µg/ml) |
|---|---|
| 0.25 | 0.115 |
| 0.5 | 0.088 |
| 1 | 0.0599 |
| 2 | 0.0325 |
| 3 | 0.0213 |
| 4 | 0.0158 |
| 5 | 0.0126 |
| 6 | 0.0114 |
| 7 | 0.0105 |
| 8 | 0.0099 |
| 9 | 0.0095 |
| 10 | 0.0091 |

A three compartment PK model is used to estimate the parameters of the PCT profile presented in Table 16. The PK model is defined as $C(t) = A*Exp(-Alpha*t) + B*Exp(-Beta*t) + C*Exp(-Gamma*t)$ where A, B, C, Alpha, Beta and Gamma are the six parameters of the model. The collected universal PK Parameters' bounds for the model parameter is given in Table 17.

Table 17

| Parameter | Unit | Lower Bound | Upper Bound |
|---|---|---|---|
| A | ug/ml | 0 | 0.52 |
| B | ug/ml | 0 | 0.52 |
| C | ug/ml | 0 | 0.52 |
| Alpha | 1/hr | 0 | 30.18 |
| Beta | 1/hr | 0 | 1.466 |
| Gamma | 1/hr | 0 | 0.15 |

User defined logical operation is division by 1 and 4 as defined by values of d = 4 and h = 1. Total $2^6 = 64$ bound combinations have been generated. Examples of some of the bound combinations are illustrated in Table 18.

Table 18

| Parameter | Unit | Lower Bound | Upper Bound 1 | Upper Bound 2 | Upper Bound 3 |
|---|---|---|---|---|---|
| A | ug/ml | 0 | 0.52 | 0.52 | 0.13 |
| B | ug/ml | 0 | 0.52 | 0.52 | 0.13 |
| C | ug/ml | 0 | 0.52 | 0.52 | 0.13 |
| Alpha | 1/hr | 0 | 30.18 | 30.18 | 7.545 |
| Beta | 1/hr | 0 | 1.466 | 0.3665 | 0.3665 |
| Gamma | 1/hr | 0 | 0.15 | 0.15 | 0.0375 |

Further, the method of the present disclosure executes the modified wind driven optimization to perform global optimization by minimizing WRSS using each of the 64 bound combinations. The globally optimized set of PK parameters for the bound combinations presented in Table 18 are illustrated in Table 19.

Table 19

| Parameter | Unit | Globally optimized Parameter 1 | Globally optimized Parameter 2 | Globally optimized Parameter 3 |
|---|---|---|---|---|
| A | ug/ml | 0.295759 | 0.25291 | 0.13 |
| B | ug/ml | 0.107908 | 0.114855 | 0.108893 |
| C | ug/ml | 0.026168 | 0.005408 | 0.005084 |
| Alpha | 1/hr | 12.67211 | 10.44327 | 5.538745 |
| Beta | 1/hr | 1.123629 | 0.351016 | 0.362702 |
| Gamma | 1/hr | 0.109171 | 0.107398 | 0.0375 |

The minimized WRSS values for above globally optimized set of PK parameters are depicted in Table 20 below:

Table 20

| | Globally optimized Parameter 1 | Globally optimized Parameter 2 | Globally optimized Parameter 3 |
|---|---|---|---|
| WRSS | 1.4E-4 | 0.00313 | 0.00609 |

Furthermore, the one or more hardware processors 104 perform a local optimization technique on the globally optimized set of PK parameters to obtain a locally optimized set of PK parameters for each of the parameter bound combination. The locally optimized set of PK parameters are used for assessing the developability of the one or more drug candidates. Here, Nelder Mead method is used for local optimization of the globally optimized set of PK parameters provided in Table 20 by minimizing WRSS. The locally optimized set of PK parameters for the globally optimized set of PK parameters in Table 19 is presented in Table 21 and corresponding minimized WRSS in Table 22.

Table 21

| Parameter | Unit | Locally Optimized parameter 1 | Locally Optimized parameter 2 | Locally Optimized parameter 3 |
|---|---|---|---|---|
| A | ug/ml | 0.315064 | 0.315064 | 0.315064 |
| B | ug/ml | 0.107594 | 0.107594 | 0.107594 |
| C | ug/ml | 0.012919 | 0.012919 | 0.01292 |
| Alpha | 1/hr | 11.49491 | 11.49492 | 11.49491 |
| Beta | 1/hr | 0.811069 | 0.81107 | 0.81107 |
| Gamma | 1/hr | 0.03486 | 0.03486 | 0.03486 |

Table 22

| | Locally Optimized parameter 1 | Locally Optimized parameter 2 | Locally Optimized parameter 3 |
|---|---|---|---|
| WRSS | 2.50021E-5 | 2.50021E-5 | 2.50021E-5 |

Further, the best set of PK parameters amongst the locally optimized set of PK parameters is selected based on the minimum of associated weighted residual sum squares (WRSS) value. Below Table 23 illustrates exemplary best set of PK parameters for all 64 bound combinations.

Table 23

| Parameter | Unit | Initial value/globally optimized value | Best Parameter/Bes t locally optimized value | Published paramete r value |
|---|---|---|---|---|
| A | ug/ml | 0.270307 | 0.315064 | 0.297 |
| B | ug/ml | 0.073894 | 0.107594 | 0.117 |

(continued)

| Parameter | Unit | Initial value/globally optimized value | Best Parameter/Best locally optimized value | Published parameter value |
|---|---|---|---|---|
| C | ug/ml | 0.010917 | 0.012919 | 0.017 |
| Alpha | 1/hr | 7.1437 | 11.49491 | 12.02 |
| Beta | 1/hr | 0.3665 | 0.81107 | 0.935 |
| Gamma | 1/hr | 0.147869 | 0.03486 | 0.064 |

The associated minimized weighted residual sum squares (WRSS) of estimated best parameters and the published parameters are 2.50E-5 and 9.42E-05 respectively. So, the method described in the present disclosure achieves a superior WRSS value indicating that the estimated PK parameters are more accurate and thus, the present disclosure offers a better solution.

A threshold-based procedure has been further introduced to get a near optimal/best PK parameter exploring a subset of all bound combinations within a limited execution time. This procedure is useful when the PK models are complex and require higher execution time and exploring all bound combination is not possible due to time constraint. Below provided is a description for the pre-defined performance threshold computation.

During the global and local optimization procedure WRSS is minimized which is the sum of weighted residuals squares and this weight varies from case studies to case studies. But, associated residual sum square (RSS) which is the sum of residual squares, is independent of weight. The formula of RSS is provided below.

$$RSS = \sum_{i=1}^{n} (C_i^{Obs} - C_i^{Pred})^2$$

Where 'n' is the number of observations in PCT profile ($C^{Obs}$, t), $C_i^{Obs}$ is the i-th value of the concentration, $C_i^{Pred}$ is i-th predicted value of the concentration.

The residual in the formula of both RSS and WRSS is the difference between observed concentration and predicted concentration and the optimization methods try to minimize it. So, it would be reasonable to consider that residual for the estimated PK parameters, to be less than a certain percentage of observed concentration. Thus, our intent here is to provide a threshold on residual sum squares (RSS) and our expectation is that the RSS for the best PK parameters should be less than the threshold. Assuming the percentage value as 'x', the proposed threshold value is computed by following formula.

$$Threshold = \sum_{i=1}^{n} \left(\frac{C_i^{Obs} * x}{100}\right)^2$$

Where 'n' is the number of observations in PCT profile ($C^{Obs}$, t)

The method further includes a plurality of steps, wherein the one or more hardware processors 104 sequentially performing the global optimization technique and the local optimization technique using the combination of multiple bounds generated from the one or more universal PK parameters' bounds to obtain the globally optimized set of PK parameters and the locally optimized set of PK parameters respectively and a best set of PK parameters is selected based on a comparison of an associated residual sum squares (RSS) value of each of the locally optimized set of PK parameters and the pre-defined performance threshold of the RSS value. The above steps of sequentially performing the global optimization technique and the local optimization technique and selecting the best set of PK parameters are better understood by way of following description:

The PCT Profile of a subject is collected from the case study PK24 of "Gabrielsson, J. & Weiner, D. Pharmacokinetic and Pharmacodynamic Data Analysis: Concepts and Applications. 4th edn. (Swedish Pharmaceutical Press, Stockholm, Sweden, 2007)" and is illustrated in Table 24. The subject was administered with constant infusion dose of 10mg/kg of a drug for 2 hours. To demonstrate the use of the method discussed above, the value of 'x' is considered as 4.8. The threshold calculation for x = 4.8 is presented in Table 24.

EP 4 510 138 A1

Table 24

| Time (hr) | Conc (μg/L) | Conc*x% | Square of Conc*x% |
|---|---|---|---|
| 0.25 | 470 | 22.56 | 508.9536 |
| 0.5 | 600 | 28.8 | 829.44 |
| 0.75 | 700 | 33.6 | 1128.96 |
| 1.05 | 750 | 36 | 1296 |
| 1.25 | 830 | 39.84 | 1587.2256 |
| 1.49 | 880 | 42.24 | 1784.2176 |
| 1.75 | 900 | 43.2 | 1866.24 |
| 1.99 | 950 | 45.6 | 2079.36 |
| 2.16 | 460 | 22.08 | 487.5264 |
| 2.35 | 330 | 15.84 | 250.9056 |
| 2.4 | 300 | 14.4 | 207.36 |
| 2.65 | 220 | 10.56 | 111.5136 |
| 2.81 | 170 | 8.16 | 66.5856 |
| 2.95 | 140 | 6.72 | 45.1584 |
| 3.11 | 120 | 5.76 | 33.1776 |
| 3.56 | 74 | 3.552 | 12.616704 |
| 4.15 | 47 | 2.256 | 5.089536 |
| 6 | 22 | 1.056 | 1.115136 |
| 7 | 17 | 0.816 | 0.665856 |
| | | Threshold =Sum (Square of Conc*x%) | 12302.1112 |

A three compartment PK differential equation models is used to estimate the parameters. The PK model is defined as

$$V_c \frac{dC_p}{dt} = Input - Cl * C_p - Cl_{d1} * C_p + Cl_{d1} * C_{t1} - Cl_{d2} * C_p + Cl_{d2} * C_{t2}$$

$$V_{t1} \frac{dC_{t1}}{dt} = Cl_{d1} * C_p - Cl_{d1} * C_{t1}$$

$$V_{t2} \frac{dC_{t2}}{dt} = Cl_{d2} * C_p - Cl_{d2} * C_{t2}$$

Where Vc, Cl, $Cl_{d1}$, $Cl_{d2}$, $V_{t1}$, and $V_{t2}$ are the parameters of the model.

The universal PK parameters' bounds of the model parameters are depicted in Table 25 below:

Table 25

| Parameter | Unit | Lower Bound | Upper Bound |
|---|---|---|---|
| Vc | L/kg | 0 | 6.3 |
| Cl | L/kg/hr | 0 | 1.904714286 |
| $Cl_{d1}$ | L/kg/hr | 0 | 1.904714286 |
| $Cl_{d2}$ | L/kg/hr | 0 | 1.904714286 |

19

(continued)

| Parameter | Unit | Lower Bound | Upper Bound |
|---|---|---|---|
| $V_{t1}$ | L/kg | 0 | 6.3 |
| $V_{t2}$ | L/kg | 0 | 6.3 |

The Logical Operation used for this model is multiplication by 1 and 4 as the user input is d = 4 and h = 1. As a result, $2^6$ = 64 bound combinations are generated, and few are depicted in Table 26.

Table 26

| Parameter | Unit | Lower Bound | Upper Bound 1 | Upper Bound 2 | Upper Bound 3 |
|---|---|---|---|---|---|
| Vc | L/kg | 0 | 6.3 | 6.3 | 6.3 |
| Cl | L/kg/hr | 0 | 1.904714286 | 1.90471 | 7.618857143 |
| $Cl_{d1}$ | L/kg/hr | 0 | 1.904714286 | 1.90471 | 7.618857143 |
| $Cl_{d2}$ | L/kg/hr | 0 | 1.904714286 | 7.61886 | 1.904714286 |
| $V_{t1}$ | L/kg | 0 | 6.3 | 6.3 | 6.3 |
| $V_{t2}$ | L/kg | 0 | 6.3 | 6.3 | 6.3 |

In threshold-based approach, the threshold is satisfied when the fifth bound combination is used for global and local optimization. The best PK parameters and related information of threshold-based approach is shown in Table 27.

Table 27

| Parameter | Lower Bound | Upper Bound | Globally Estimated Value | Locally Estimated Value/Best Parameters |
|---|---|---|---|---|
| Vc | 0 | 6.3 | 0.77578 | 0.765425 |
| Cl | 0 | 1.90471 | 1.904714 | 5.094639 |
| $Cl_{d1}$ | 0 | 1.90471 | 0.756249 | 6.567688 |
| $Cl_{d2}$ | 0 | 7.61886 | 0.46402 | 0.724231 |
| $V_{t1}$ | 0 | 6.3 | 4.737196 | 1.521465 |
| $V_{t2}$ | 0 | 6.3 | 6.024058 | 2.375013 |

Estimated Parameters exploring all the bound combinations is shown in Table 28 below:

Table 28

| Parameter | Globally Estimated Value | Locally Estimated Value | Published Parameter value |
|---|---|---|---|
| Vc | 1.931949 | 0.757642 | 0.801475 |
| Cl | 5.210796 | 5.093557 | 5.09876 |
| $Cl_{d1}$ | 0.692709 | 6.658893 | 6.328982 |
| $Cl_{d2}$ | 1.661407 | 0.723764 | 0.722152 |
| $V_{t1}$ | 3.034314 | 1.526124 | 1.495898 |
| $V_{t2}$ | 0.799883 | 2.37912 | 2.397024 |

The comparative results of threshold-based approach and exploring all bound combination is depicted in Table 29 below.

Table 29

|  | RSS | WRSS | Combinations considered | Execution Time (min) |
|---|---|---|---|---|
| Threshold-based approach | 11839.60755 | 0.0219 | 5 | 12 |
| All Bound Combinations | 11759.16841 | 0.0219 | 64 | 164 |
| Published Value |  | 0.022 |  |  |

It is observed that the execution time of threshold-based approach is approximately 13 times faster and the similar trend is observed in other case studies. Based on the comparison of WRSS and parameter values of the two approaches and execution time, it is demonstrated that the threshold-based approach is an efficient solution particularly, when execution time is a constraint.

The above experiments were executed with a system configuration of: Processor: Intel(R) Core (TM) i5-10310U CPU @ 1.70GHz 2.21GHz, RAM: 16GB, and System type: 64-bit operating system, x64- based processor.

Embodiments of the present disclosure provide systems and methods for generating optimized (best) set of PK parameter particularly, by a) creating universal PK parameters' bounds, b) performing logical operations on universal PK parameters' bounds to create multiple bound combinations c) computing a performance threshold for residual sum of squares (RSS) d) performing global optimization to estimate globally optimized set of PK parameters to be used as initial PK parameters and e) performing local optimization on initial PK parameters to estimate locally optimized set of PK parameters to be considered as best PK parameters The method enables defining the PK parameter range for PK parameter estimation, thereby reducing redundant computations or low-quality potential PK parameters being estimated and further enabling exploration of the search region effectively.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.  A processor implemented method comprising:

     receiving, via one or more hardware processors, values of a plurality of pharmacokinetic (PK) parameters pertaining drug candidates (202);
     preprocessing, via the one or more hardware processors, the values of the plurality of PK parameters to obtain preprocessed values of the plurality of PK parameters, in accepted units (204);
     assigning, via the one or more hardware processors, a lower bound to each of the plurality of PK parameters (206);
     determining, via the one or more hardware processors, an upper bound for each of the plurality of PK parameters (208), wherein the step of determining the upper bound for each of the plurality of PK parameters comprises:

          arranging and sorting of the preprocessed values of the plurality of PK parameters in a pre-defined order to obtain a sorted list of values of the PK parameters;
          computing one or more percentiles values from the sorted list of values of the PK parameters;
          testing usefulness of the one or more computed percentile values as one or more upper bounds; and
          determining a percentile value amongst the one or more percentile values as the upper bound;

     identifying, via the one or more hardware processors, one or more universal PK parameters' bounds further comprising one or more parameter names, the lower bound, the upper bound and the accepted units (210);
     performing, via the one or more hardware processors, a global optimization technique on the plurality of pharmacokinetic (PK) parameters using the one or more universal PK parameters' bounds and a plasma-concentration time (PCT) profile of the one or more drug candidates to obtain a globally optimized set of PK parameters for a given PK model (212); and
     performing, via the one or more hardware processors, a local optimization technique on the globally optimized set of PK parameters to obtain a locally optimized set of PK parameters, wherein the locally optimized set of PK parameters are used for assessing the developability of the one or more drug candidates (214).

2.  The processor implemented method as claimed in claim 1, wherein the globally optimized set of PK parameters is obtained by:

     dynamically generating a combination of multiple bounds from the one or more universal PK parameters' bounds, using a logical operation technique on the one or more universal PK parameters' bounds, the automatic logical operation technique comprises:

          performing one or more logical operations on the upper bound of the one or more universal PK parameters' bounds of the plurality of PK parameters; and
          generating one or more upper bound combinations based on one or more values obtained after logical operations for each of the plurality of PK parameters to obtain the combination of multiple bounds further comprising upper bound combinations and zero as lower bound for all of the combinations; and

     estimating PK parameters of the given PK model from the combination of multiple bounds using the global optimization technique, wherein the estimated PK parameters serve as the globally optimized set of PK parameters.

3.  The processor implemented method as claimed in claim 2, wherein a best set of PK parameters amongst the locally optimized set of PK parameters is obtained by:

for each globally optimized set of PK parameters of the given PK model from the combination of multiple bounds, performing the local optimization technique on the globally optimized set of PK parameters to obtain the locally optimized set of PK parameters,
wherein the best set of PK parameters amongst the locally optimized set of PK parameters is selected based on an associated weighted residual sum squares (WRSS) value.

4. The processor implemented method as claimed in claim 2, wherein the global optimization technique and the local optimization technique are performed on the combination of multiple bounds based on a pre-defined performance threshold of a residual sum squares (RSS) value.

5. The processor implemented method as claimed in claim 4, further comprising:

sequentially performing the global optimization technique and the local optimization technique for the combination of multiple bounds generated from the one or more universal PK parameters' bounds to obtain the locally optimized set of PK parameters; and
selecting a best set of PK parameters based on a comparison of an associated residual sum squares (RSS) value of each of the locally optimized set of PK parameters and the pre-defined performance threshold of the RSS value.

6. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive values of a plurality of pharmacokinetic (PK) parameters pertaining to drug candidates;
preprocess the values of the plurality of PK parameters to obtain preprocessed values of the plurality of PK parameters, in accepted units;
assign a lower bound to each of the plurality of PK parameters;
determine an upper bound for each of the plurality of PK parameters, wherein the step of determining the upper bound for each of the plurality of PK parameters comprises:

arranging and sorting the preprocessed values of the plurality of PK parameters in a pre-defined order to obtain a sorted list of values of the PK parameters;
computing one or more percentiles values from the sorted list of values of the PK parameters;
testing usefulness of the one or more computed percentile values as one or more upper bounds; and
determining a percentile value amongst the one or more percentile values as the upper bound;

identifying one or more universal PK parameters' bounds further comprising one or more parameter names, the lower bound, the upper bound and the accepted units;
performing a global optimization technique on the plurality of pharmacokinetic (PK) parameters using the one or more universal PK parameters' bounds and a plasma-concentration time (PCT) profile of the one or more drug candidates to obtain a globally optimized set of PK parameters for a given PK model; and
performing a local optimization technique on the globally optimized set of PK parameters to obtain a locally optimized set of PK parameters, wherein the locally optimized set of PK parameters are used for assessing the developability of the one or more drug candidates.

7. The system as claimed in claim 6, wherein the globally optimized set of PK parameters is obtained by:

dynamically creating a combination of multiple bounds generated from the one or more universal PK parameters' bounds, using a logical operation technique on the one or more universal PK parameters' bounds, the automatic logical operation technique comprises:

performing one or more logical operations on the upper bound of the one or more universal PK parameters' bounds of the plurality of PK parameters; and
generating one or more upper bound combinations based on one or more values obtained after logical operations for each of the plurality of PK parameters to obtain the combination of multiple bounds further comprising upper bound combinations and zero as lower bound for all of the combinations; and

estimating PK parameters of the given PK model from the combination of multiple bounds using the global optimization technique, wherein the estimated PK parameters serve as the globally optimized set of PK parameters.

8. The system as claimed in claim 7, wherein a best set of PK parameters amongst the locally optimized set of PK parameters is obtained by:

   for each globally optimized PK parameter of the given PK model from the combination of multiple bounds, performing the local optimization technique on the globally optimized set of PK parameters to obtain the locally optimized set of PK parameters,
   wherein the best set of PK parameters amongst the locally optimized set of PK parameters is selected based on an associated weighted residual sum squares (WRSS) value.

9. The system as claimed in claim 7, wherein the global optimization technique and the local optimization technique are performed on the combination of multiple bounds based on a pre-defined performance threshold of a residual sum squares (RSS) value.

10. The system as claimed in claim 9, wherein the one or more hardware processors are further configured by the instructions to:

    sequentially perform the global optimization technique and the local optimization technique for the combination of multiple bounds generated from the one or more universal PK parameters' bounds to obtain the locally optimized set of PK parameters; and
    select a best set of PK parameters based on a comparison of an associated residual sum squares (RSS) value of each of the locally optimized set of PK parameters and the pre-defined performance threshold of the RSS value.

11. One or more non-transitory machine-readable information storage mediums comprising one or more instructions which when executed by one or more hardware processors cause:

    receiving values of a plurality of pharmacokinetic (PK) parameters pertaining drug candidates;
    preprocessing the values of the plurality of PK parameters to obtain preprocessed values of the plurality of PK parameters, in accepted units;
    assigning a lower bound to each of the plurality of PK parameters;
    determining an upper bound for each of the plurality of PK parameters, wherein the step of determining the upper bound for each of the plurality of PK parameters comprises:

       arranging and sorting of the preprocessed values of the plurality of PK parameters in a pre-defined order to obtain a sorted list of values of the PK parameters;
       computing one or more percentiles values from the sorted list of values of the PK parameters;
       testing usefulness of the one or more computed percentile values as one or more upper bounds; and
       determining a percentile value amongst the one or more percentile values as the upper bound;

    identifying one or more universal PK parameters' bounds further comprising one or more parameter names, the lower bound, the upper bound and the accepted units;
    performing a global optimization technique on the plurality of pharmacokinetic (PK) parameters using the one or more universal PK parameters' bounds and a plasma-concentration time (PCT) profile of the one or more drug candidates to obtain a globally optimized set of PK parameters for a given PK model; and
    performing a local optimization technique on the globally optimized set of PK parameters to obtain a locally optimized set of PK parameters, wherein the locally optimized set of PK parameters are used for assessing the developability of the one or more drug candidates.

12. The one or more non-transitory machine-readable information storage mediums as claimed in claim 11, wherein the globally optimized set of PK parameters is obtained by:

    dynamically generating a combination of multiple bounds from the one or more universal PK parameters' bounds, using a logical operation technique on the one or more universal PK parameters' bounds, the automatic logical operation technique comprises:

performing one or more logical operations on the upper bound of the one or more universal PK parameters' bounds of the plurality of PK parameters; and

generating one or more upper bound combinations based on one or more values obtained after logical operations for each of the plurality of PK parameters to obtain the combination of multiple bounds further comprising upper bound combinations and zero as lower bound for all of the combinations; and

estimating PK parameters of the given PK model from the combination of multiple bounds using the global optimization technique, wherein the estimated PK parameters serve as the globally optimized set of PK parameters.

13. The one or more non-transitory machine-readable information storage mediums as claimed in claim 12, wherein a best set of PK parameters amongst the locally optimized set of PK parameters is obtained by:

for each globally optimized set of PK parameters of the given PK model from the combination of multiple bounds, performing the local optimization technique on the globally optimized set of PK parameters to obtain the locally optimized set of PK parameters, wherein the best set of PK parameters amongst the locally optimized set of PK parameters is selected based on an associated weighted residual sum squares (WRSS) value.

14. The one or more non-transitory machine-readable information storage mediums as claimed in claim 12, wherein the global optimization technique and the local optimization technique are performed on the combination of multiple bounds based on a pre-defined performance threshold of a residual sum squares (RSS) value.

15. The one or more non-transitory machine-readable information storage mediums as claimed in claim 14, wherein the one or more instructions which when executed by the one or more hardware processors further cause:

sequentially performing the global optimization technique and the local optimization technique for the combination of multiple bounds generated from the one or more universal PK parameters' bounds to obtain the locally optimized set of PK parameters; and

selecting a best set of PK parameters based on a comparison of an associated residual sum squares (RSS) value of each of the locally optimized set of PK parameters and the pre-defined performance threshold of the RSS value.

SYSTEM
100

MEMORY
102

DATABASE
108

HARDWARE
PROCESSOR(S)
104

INTERFACE(S)
106

**FIG. 1**

```
┌─────────────────────────────────────────────┐
│  receiving values of a plurality of pharmacokinetic (PK)  │── 202
│  parameters (PPP) pertaining to one or more drug candidates │
└─────────────────────────────────────────────┘
                      │
                      ▼
┌─────────────────────────────────────────────┐
│  preprocessing the value of plurality of PK parameters to  │
│  obtain preprocessed values of the plurality of PK        │── 204
│  parameters, in accepted units                            │
└─────────────────────────────────────────────┘
                      │
                      ▼
┌─────────────────────────────────────────────┐
│  assigning a lower bound to each of the PPP               │── 206
└─────────────────────────────────────────────┘
                      │
                      ▼
┌─────────────────────────────────────────────┐
│  determining an upper bound for each of the PPP, the step of │
│  determining the upper bound for each of the PPP comprises:  │
│  arranging and sorting each of the preprocessed values of   │
│  the PPP in a pre-defined order to obtain a sorted list of   │
│  values of the PPP;                                         │── 208
│  computing one or more percentiles values from the sorted   │
│  list of values of the PK parameters;                      │
│  testing a usefulness of the one or more computed percentile │
│  values as one or more upper bounds; and                    │
│  determining a percentile value amongst the one or more     │
│  percentile values as the upper bound                       │
└─────────────────────────────────────────────┘
                      │
                      ▼
┌─────────────────────────────────────────────┐
│  identifying universal PK parameters' bounds comprising    │
│  parameter names, the lower bound, the upper bound and     │── 210
│  the accepted units                                        │
└─────────────────────────────────────────────┘
                      │
                      ▼
┌─────────────────────────────────────────────┐
│  performing a global optimization technique on the plurality of │
│  pharmacokinetic (PK) parameters using the universal PK        │
│  parameters' bounds and a plasma-concentration time (PCT)      │── 212
│  profile of the one or more drug candidates to obtain a        │
│  globally optimized set of PK parameters for a given PK        │
│  model                                                        │
└─────────────────────────────────────────────┘
                      │
                      ▼
┌─────────────────────────────────────────────┐
│  performing a local optimization technique on the globally     │
│  optimized set of PK parameters to obtain a locally optimized  │
│  set of PK parameters, wherein the locally optimized set of    │── 214
│  PK parameters are used for assessing the developability of    │
│  the one or more drug candidates                              │
└─────────────────────────────────────────────┘
```

**FIG. 2**

Europäisches
Patentamt

European
Patent Office

Office européen
des brevets

# EUROPEAN SEARCH REPORT

**Application Number**

EP 24 15 3674

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NARAYANAN A. N. ET AL: "An Efficient Methodology for PK-PD Data Analysis Based on Novel Bound Selection and Modified Spiral Dynamic Optimization Methods", 2021 IEEE INTERNATIONAL CONFERENCE ON BIOINFORMATICS AND BIOMEDICINE (BIBM), IEEE, 9 December 2021 (2021-12-09), pages 259-265, XP034066425, DOI: 10.1109/BIBM52615.2021.9669389 [retrieved on 2022-01-04] * the whole document * | 1-15 | INV. G16C20/30 |
| X | US 2021/050108 A1 (RAMAMURTHI NARAYANAN [IN] ET AL) 18 February 2021 (2021-02-18) * the whole document * | 1-15 | |
| X | US 2015/269225 A1 (AGRAWAL NISHANT KUMAR [IN] ET AL) 24 September 2015 (2015-09-24) * the whole document * | 1-15 | |
| X | DAS S. S. ET AL: "Hybrid Modified League Championship Algorithm and its Applications in Compartmental Pharmacokinetic-Pharmacodynamic Data Analysis", 2022 IEEE INTERNATIONAL CONFERENCE ON BIOINFORMATICS AND BIOMEDICINE (BIBM), IEEE, 6 December 2022 (2022-12-06), pages 3248-3251, XP034264904, DOI: 10.1109/BIBM55620.2022.9995090 [retrieved on 2023-01-02] * the whole document * | 1-15 | **TECHNICAL FIELDS SEARCHED (IPC)** G16C |

The present search report has been drawn up for all claims

| Place of search | Date of completion of the search | Examiner |
|---|---|---|
| The Hague | 19 July 2024 | Godzina, Przemyslaw |

EPO FORM 1503 03.82 (P04C01)

## ANNEX TO THE EUROPEAN SEARCH REPORT
## ON EUROPEAN PATENT APPLICATION NO.

EP 24 15 3674

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2024

| Patent document cited in search report | Publication date | Patent family member(s) | Publication date |
|---|---|---|---|
| US 2021050108 A1 | 18-02-2021 | EP 3786962 A1 | 03-03-2021 |
| | | US 2021050108 A1 | 18-02-2021 |
| US 2015269225 A1 | 24-09-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321054496

**Non-patent literature cited in the description**

- **OBACH, R. SCOTT** ; **FRANCO LOMBARDO** ; **NIGEL J. WATERS.** Trend analysis of a database of intravenous pharmacokinetic parameters in humans for 670 drug compounds. *Drug Metabolism and Disposition*, 2008, vol. 36 (7), 1385-1405
- **BOWER, SUSANNE** ; **C. J. HULL**. Comparative pharmacokinetics of fentanyl and alfentanil.. *British Journal of Anaesthesia*, 1982, vol. 54 (8), 871-877
- **RIPA, S.** ; **L. FERRANTE,** ; **M. PRENNA.** A linear model for the pharmacokinetics of azithromycin in healthy volunteers.. *Chemotherapy*, 1996, vol. 42 (6), 402-409
- **BARNETT, GENE** ; **RICHARD HAWKS** ; **RICHARD RESNICK**. Cocaine pharmacokinetics in humans.. *Journal of ethnopharmacology*, 1981, vol. 3 (2-3), 353-366
- **RIPA, S.** ; **L. FERRANTE** ; **M. PRENNA.** A linear model for the pharmacokinetics of azithromycin in healthy volunteers.. *Chemotherapy*, 1996, vol. 42 (6), 402-409
- **STAGNI, GRAZIA** ; **PATRICK J. DAVIS** ; **THOMAS M. LUDDEN**. Human pharmacokinetics of betaxololenantiomers.. *Journal of pharmaceutical sciences*, 1991, vol. 80 (4), 321-324

- **GRIMALDI, R. et al.** Pharmacokinetic and pharmacodynamic studies following the intravenous and oral administration of the antiparkinsonian drug biperiden to normal subjects.. *European journal of clinical pharmacology*, 1986, vol. 29 (6), 735-737
- **CUNDY, KENNETH C. et al.** Clinical pharmacokinetics of adefovir in human immunodeficiency virus type 1-infected patients.. *Antimicrobial agents and chemotherapy*, 1995, vol. 39 (11), 2401-2405
- **STAPLETON, STACIE L. et al.** Plasma and cerebrospinal fluid pharmacokinetics of pemetrexed after intravenous administration in non-human primates.. *Cancer chemotherapy and pharmacology*, 2007, vol. 59 (4), 461-466
- **VAN HAMME** ; **MICHAEL J.** ; **M. M. GHONEIM** ; **JOHN J. AMBRE**. Pharmacokinetics of etomidate, a new intravenous anesthetic.. *Anesthesiology*, 1978, vol. 49 (4), 274-277
- Pharmacokinetic and Pharmacodynamic Data Analysis. **GABRIELSSON, J.** ; **WEINER, D.** Concepts and Applications. Swedish Pharmaceutical Press, 2007